**Europäisches Patentamt**

**European Patent Office**

**Office européen des brevets**

(19)

(11) Veröffentlichungsnummer: **0 155 590**
A2

(12) # EUROPÄISCHE PATENTANMELDUNG

(21) Anmeldenummer: **85102499.2**

(22) Anmeldetag: **06.03.85**

(51) Int. Cl.⁴: **C 12 N 15/00**
C 12 N 1/20
//(C12N1/20, C12R1:19)

(30) Priorität: **19.03.84 DE 3409966**

(43) Veröffentlichungstag der Anmeldung:
**25.09.85 Patentblatt 85/39**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Engels, Joachim, Dr.**
**Feldbergstrasse 1**
**D-6242 Kronberg/Taunus(DE)**

(72) Erfinder: **Leineweber, Michael, Dr.**
**Heimchenweg 74**
**D-6230 Frankfurt am Main 80(DE)**

(72) Erfinder: **Uhlmann, Eugen, Dr.**
**Washington Street 287**
**Belmont, Mass. 02178(US)**

(72) Erfinder: **Ulmer, Wolfgang, Dr.**
**Dr. Fuchs-Strasse 8**
**D-6238 Friedrichsdorf(DE)**

(54) **Gentechnologisches Verfahren zur Herstellung von Human-Gamma-interferon und Mitter zur Durchfürung dieses Verfahrens.**

(57) Mit Hilfe einer speziellen DNA-Sequenz kann in einem gentechnologischen Verfahren Human-γ-Interferon gewonnen werden. Das Gen wird vorteilhaft in Form mehrerer Fragmente synthetisiert, die enzymatisch zu größeren Teilsequenzen ligiert werden, welche in Hybridplasmide eingebaut und in Wirtsorganismen amplifiziert werden. Nach Reisolierung der Teilsequenzen werden diese zum Gesamtgen vereinigt, in ein Hybridplasmid eingebaut und dieses in einem Wirtsorganismus zur Expression gebracht.

EP 0 155 590 A2

_ 1 _

Gentechnologisches Verfahren zur Herstellung von Human-Gammainterferon und Mittel zur Durchführung dieses Verfahrens

Die Erfindung betrifft ein Verfahren zur Herstellung von Human-Gammainterferon, chemisch synthetisierte Gene, die dieses Peptid codieren sowie geeignete Vektorkonstruktionen und Wirtsorganismen zur Expression dieses Polypeptides.

Das Gammainterferon (früher Immuninterferon oder Typ II-Interferon; hier kurz als IFN-γ bezeichnet) wurde im Jahre 1965 von F. Wheelock entdeckt (Wheelock; Science 149 (1965), 310), welcher zeigte, daß IFN-γ bestimmte Zellen vor einer Virusinfektion schützen kann. Menschliches IFN-γ (Basiswissen s. W.E. Stewart, II, The Interferon System, Springer Verlag (2nd ed., 1981)) ist ein Polypeptid aus 146 Aminosäuren (Gray et al., Nature 295 (1982), 503), das von Natur aus glykosyliert ist. Das Glykoprotein besitzt ein Molekulargewicht von etwa 63 000 - 73 000 (Pestka et al., J. Biol. Chem. 258 (1983), 9706) und liegt in seiner funktionellen Form wahrscheinlich als Tetramer vor. Die Glykosylierung des IFN-γ ist für seine Funktionalität nicht erforderlich; so reduziert die Glykosidase-Behandlung von IFN-γ nicht seine antivirale Aktivität in humanen Fibroblasten-Zellkulturen (Keller et al., J. Biol. Chem. 258 (1983), 8010).

Ferner ist IFN-γ im Gegensatz zu den Alphainterferonen und Betainterferon bei pH 2 instabil und wird auch durch Hitze (60°C ) desaktiviert.

Die Gewinnung von Human-IFN-γ aus Zellkulturen menschlicher Zellinien oder aus Leukozyten (Blutkonserven) ist nur in schlechter Ausbeute und niedriger Produktreinheit möglich. Es wurden deshalb schon gentechnologische Verfahren zur Herstellung von IFN-γ-ähnlichen Polypeptiden beschrieben, beispielsweise in der Europäischen Patentanmeldung mit der Veröffentlichungsnummer 95 350 die chemische Synthese eines für IFN-γ codierenden Gens, dessen Einbau in ein Hybridplasmid, die Transformation von E. coli und die Expression eines immunologisch aktiven Polypeptids.

Human-IFN-γ besitzt folgende Peptidsequenz (Devos et al., Nucl. Acids Research <u>10</u> (1982), 2487):

$Cys^1$ – Tyr – Cys – Gln – Asp – Pro – Tyr – Val – Lys – $Glu^{10}$–
Ala – Glu – Asn – Leu – Lys – Lys – Tyr – Phe – Asn – $Ala^{20}$–
Gly – His – Ser – Asp – Val – Ala – Asp – Asn – Gly – $Thr^{30}$–
Leu – Phe – Leu – Gly – Ile – Leu – Lys – Asn – Trp – $Lys^{40}$–
Glu – Glu – Ser – Asp – Arg – Lys – Ile – Met – Gln – $Ser^{50}$–
Gln – Ile – Val – Ser – Phe – Tyr – Phe – Lys – Leu – $Phe^{60}$–
Lys – Asn – Phe – Lys – Asp – Asp – Gln – Ser – Ile – $Gln^{70}$–
Lys – Ser – Val – Glu – Thr – Ile – Lys – Glu – Asp – $Met^{80}$–
Asn – Val – Lys – Phe – Phe – Asn – Ser – Asn – Lys – $Lys^{90}$–
Lys – Arg – Asp – Asp – Phe – Glu – Lys – Leu – Thr – $Asn^{100}$–
Tyr – Ser – Val – Thr – Asp – Leu – Asn – Val – Gln – $Arg^{110}$–
Lys – Ala – Ile – His – Glu – Leu – Ile – Gln – Val – $Met^{120}$–
Ala – Glu – Leu – Ser – Pro – Ala – Ala – Lys – Thr – $Gly^{130}$–
Lys – Arg – Lys – Arg – Ser – Gln – Met – Leu – Phe – $Arg^{140}$–
Gly – Arg – Arg – Ala – Ser – $Gln^{146}$.

Die vorliegende Erfindung betrifft die gentechnologische Herstellung von Human-IFN-γ mit Hilfe eines chemisch synthetisierten Gens, das durch die DNA-Sequenz I (Anhang) gekennzeichnet ist.

Der genetische Code ist bekanntlich "entartet", d.h. daß nur für zwei Aminosäuren eine einzige Nucleotid-Sequenz codiert, während den restlichen 18 genetisch codierbaren Aminosäuren 2 bis 6 Tripletts zuzuordnen sind. Von den hierdurch gegebenen Variationsmöglichkeiten machen außerdem die Wirtszellen unterschiedlicher Spezies nicht immer den gleichen Gebrauch. Für die Synthese der Gene besteht somit eine unübersehbare Vielfalt von Codon-Möglichkeiten. Es wurde nun gefunden, daß die DNA-Sequenz I (Anhang), die für die gesamte Aminosäurensequenz 1-146 codiert, sowie die zur Synthese der Sequenz I benutzten DNA-Teilsequenzen besonders vorteilhaft für die gentechnologische Synthese von IFN-γ sind. An das 5'-Ende des codierenden Stranges der DNA-Sequenz I schließt sich eine "überhängende" DNA-Sequenz an, beispielsweise entsprechend der Restriktionsendonuclease Eco RI, am 3'-Ende des codierenden Stranges dagegen eine einzelsträngige, vorteilhaft von der am 5'-Ende verschiedene überhängende Sequenz, beispielsweise entsprechend dem Restriktionsenzym Sal I. Diese beiden unterschiedlichen Erkennungssequenzen gewährleisten die Insertion der DNA in Plasmide in der gewünschten Orientierung. Man kann auch gleiche überhängende Sequenzen wählen und später eine entsprechende Selektion vornehmen.

Zwischen diesen Erkennungssequenzen und den Codons für die Aminosäurefolge befindet sich am 5'-Ende des codierenden Stranges das Codon für die Aminosäure Methionin. Alternativ hierzu kann eine Praesequenz (auch Signal- oder leader-Sequenz genannt) eines bakteriellen oder sonstigen wirtseigenen Proteins stehen (Übersichtsartikel: Perlman und Halvorson; J. Mol. Biol. 167 (1983), 391), welche beispielsweise die Sekretion des gewünschten Polypeptids aus dem Cytoplasma bewirkt und bei diesem Exkretionsprozeß von einer in der Wirtszelle natürlich vorkommenden Signal-Peptidase abgespalten wird. Im Anschluß an das für Glutamin codierende Triplett 146 folgen dann ein Stop-Codon oder vorzugsweise zwei Stop-Tripletts.

Zwei interne singuläre Schnittstellen für die Restriktionsenzyme Bam HI bzw. Hind III (im Codon 31 bzw. 94 des codierenden Stranges oder im Codon 32 bzw. 95 des nichtcodierenden Strangs) ermöglichen die Subklonierung dreier Genfragmente IFN-I, IFN-II und IFN-III (siehe DNA-Sequenz II), die in gut untersuchte Klonierungsvektoren, wie etwa pBR 322 oder pUC 8, eingebaut werden können. Zusätzlich wurden innerhalb des Strukturgens eine Reihe von weiteren singulären Erkennungssequenzen für Restriktionsenzyme eingebaut, die einerseits einen Zugang zu Teilsequenzen des IFN-$\gamma$ schaffen und andererseits die Durchführung von Variationen erlauben:

| Restriktionsenzym | Schnitt nach Nucleotid-Nr. (codierender Strang) | |
| --- | --- | --- |
| | in Sequenz I | in Sequenz II |
| Ava II[a] | 12 | 20 |
| Alu I[b] | 31 | 39 |
| Hinf I[a] | 126 | 134 |
| Dde I[c] | 151 | 159 |
| Aha III[c] | 191 | 199 |
| Taq I[c] | 286 | 294 |
| Aha III[d] | 319 | 327 |
| Sst I[a] | 349 | 357 |
| Bst NI[d] | 354 | 362 |
| Pst I[a] | 380 | 388 |
| Bbv I[a] | 390 | 398 |
| Sst II[a] | 422 | 430 |
| Dde I[d] | 436 | 444 |

a) singulär bezüglich der Gesamt-DNA-Sequenz I
b) singulär bezüglich der Teilsequenz IFN-I
c) singular bezüglich der Teilsequenz IFN-II
d) singulär bezüglich der Teilsequenz IFN-III

Das Gen, bestehend aus der DNA-Sequenz I, dem Codon für Methionin und den überstehenden Enden läßt sich aus 34 Oligonucleotiden mit einer Länge von 18 bis 33 Nucleotiden aufbauen (siehe DNA-Sequenz II), indem diese zunächst chemisch synthetisiert und dann über "sticky ends" von 4 bis 6 Nucleotiden enzymatisch verknüpft werden.

Bei der DNA-Sequenz I wurde weiterhin berücksichtigt, daß bei denjenigen Aminosäuren, denen mehrere Codons zuzuordnen sind, diese nicht gleichwertig sind, sondern vielmehr in der jeweiligen Wirtszelle wie E. coli unterschiedliche Präferenzen zeigen. Weiterhin wurden palindromische Sequenzen auf ein Mindestmaß reduziert.

Die Genstruktur der DNA-Sequenz I ist somit leicht aus relativ kleinen Bausteinen zugänglich, ermöglicht die Subklonierung dreier Genfragmente in gut bekannte Vektoren und erlaubt deren Kombination zum Gesamtgen sowie gegebenenfalls Veränderungen derselben.

Der Einbau der synthetischen Gene bzw. Genfragmente in Klonierungsvektoren, beispielsweise in die handelsüblichen Plasmide pUC 8 und pBR 322 bzw. andere allgemein zugängliche Plasmide wie ptac 11 und pKK 177.3, erfolgt in an sich bekannter Weise. Auch können die chemisch synthetisierten Gene zuvor mit geeigneten chemisch synthetisierten Kontrollregionen versehen werden, die eine Expression der Proteine ermöglichen. Hierzu kann auf das Lehrbuch von Maniatis (Molecular Cloning, Maniatis et al., Cold Spring Harbor, 1982) verwiesen werden. Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben. Die Gewinnung des exprimierten Proteins und dessen Reinigung sind ebenfalls beschrieben (J.A. Georgiades, Texas Reports in Biology and Medicine 41 (1981) 179; Came and Carter (Editors), "Interferons and Their Applications", Springer-Verlag 1984).

Die erfindungsgemäß erhaltenen Genfragmente IFN-I, IFN-II und IFN-III, die damit erhaltenen Hybridplasmide und die transformierten Wirtsorganismen sind neu und Gegenstand der Erfindung. Dasselbe gilt für aus der DNA-Sequenz I abgewandelte neue DNA-Sequenzen. Weitere Ausgestaltungen der Erfindung sind in den Patentansprüchen niedergelegt.

In den folgenden Beispielen werden noch einige Ausgestaltungen der Erfindung im einzelnen erläutert, woraus sich die Vielzahl der möglichen Abwandlungen und Kombinationen für den Fachmann ergeben. Prozentangaben beziehen sich hierbei auf das Gewicht, wenn nichts anderes angegeben ist.

Beispiele
1. Chemische Synthese eines einzelsträngigen Oligonucleotids

Am Beispiel des Genbausteins Ia, der die Nucleotide 1-23 des codierenden Strangs umfaßt, wird die Synthese der Genbausteine erläutert. Nach bekannten Methoden (M.J. Gait et al., Nucleic Acids Res. 8 (1980) 1081-1096)) wird das am 3'-Ende stehende Nucleosid, im vorliegenden Falle also Cytidin (Nucleotid Nr. 23), an Kieselgel ($^{(R)}$FRACTOSIL, Firma Merck) über die 3'-Hydroxyfunktion covalent gebunden. Hierzu wird zunächst das Kieselgel unter Abspaltung von Ethanhol mit 3-(Triethoxysilyl)-propylamin umgesetzt, wobei eine Si-O-Si-Bindung entsteht. Das Cytidin wird als $N^4$-Benzoyl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylaminogruppe acyliert.

In den folgenden Syntheseschritten wird die Basenkomponente als 5'-O-Dimethoxytrityl-nucleosid-3'-phosphorigsäuremonomethylester-dialkylamid oder -chlorid eingesetzt, wobei das Adenin als $N^6$-Benzoyl-Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

50 mg des polymeren Trägers, der 2 µmol Cytosin gebunden enthält, werden nacheinander mit den folgenden Agentien behandelt:

a) Nitromethan,

b) gesättigte Zinkbromidlösung in Nitromethan mit 1 % Wasser,

c) Methanol,

d) Tetrahydrofuran,

e) Acetonitril,

f) 40 µmol des entsprechenden Nucleosidphosphits und 200 µmol Tetrazol in 0,5 ml wasserfreiem Acetonitril (5 Minuten),

g) 20 % Acetanhydrid in Tetrahydrofuran mit 40 % Lutidin und 10 % Dimethylaminopyridin (2 Minuten),

h) Tetrahydrofuran,

i) Tetrahydrofuran mit 20 % Wasser und 40 % Lutidin,

j) 3 % Jod in Kollidin/Wasser/Tetrahydrofuran im Volumenverhältnis 5:4:1,

k) Tetrahydrofuran und

l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion b) spektrophotometrisch durch Messung der Absorption des Dimethoxytritylkations bei einer Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten.

Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib-IIIl synthetisiert, deren Nucleotidfolge aus der DNA-Sequenz II hervorgeht.

2. Enzymatische Verknüpfung der einzelsträngigen Oligonucleotide zu den Genfragmenten IFN-I, IFN-II und IFN-III

Zur Phosphorylierung der Oligonucleotide am 5'-Terminus wurde je 1 nmol der Oligonucleotide Ia und Ib mit 5 nmol Adenosintriphosphat mit vier Einheiten T4-Polynucleotid-Kinase in 20 µl 50 mM Tris-HCl-Puffer (pH 7,6), 10 mM Magnesiumchlorid und 10 mM Dithiothreitol (DTT) 30 Minuten bei 37°C behandelt (C.C. Richardson, Progress in Nucl. Acids Res. 2 (1972) 825). Das Enzym wird durch fünfminütiges Erhitzen auf 95°C desaktiviert. Anschließend werden die Oligonucleotide Ia und Ib gegeneinander hybridisiert, indem man sie in wäßriger Lösung 2 Minuten auf 95°C erhitzt und dann langsam auf 5°C abkühlt.

Analog werden die Oligonucleotide Ic und Id, Ie und If, Ig und Ih sowie Ii und Ij phosphoryliert und paarweise hybridisiert. Für das Subfragment IFN-II werden die Oligonucleotide IIa mit IIb usw. bis IIk mit IIl und für das Subfragment IFN-III die Oligomeren IIIa mit IIIb usw. bis IIIk mit IIIl phosphoryliert und paarweise hybridisiert.

Die so erhaltenen fünf Oligonucleotidpaare für das Genfragment IFN-I. bzw. die sechs Oligonucleotidpaare für die Genfragmente IFN-II und IFN-III werden jeweils wie folgt ligiert:

Die doppelsträngigen Nucleotide werden vereinigt und in jeweils 40 μl 50 mM Tris-HCl-Puffer, 20 mM Magnesiumchlorid und 10 mM DTT mit Hilfe von 100 Einheiten. T4-DNA-Ligase bei 15°C im Laufe von 16 Stunden ligiert.

Die Reinigung der Genfragmente IFN-I bis IFN-III erfolgt durch Gelelektrophorese auf einem 10%igen Polyacrylamidgel (ohne Harnstoffzusatz, 20 · 40 cm, 1 mm Dicke), wobei als Markersubstanz ØX 174 DNA (Fa. BRL), geschnitten mit Hinf I, oder pBR 322, geschnitten mit Hae III, diente.

3. Herstellung von Hybridplasmiden, die die Genfragmente IFN-I, IFN-II und IFN-III enthalten

a) Einbau des Genfragmentes IFN-I in pBR 322
Das handelsübliche Plasmid pBR 322 wird in bekannter Weise mit den Restriktionsendonucleasen Eco RI und

- 10 -                                    0155590

Bam HI nach den Angaben der Hersteller geöffnet.
Der Verdauungsansatz wird auf einem 5%igen Polyacrylamidgel durch Elektrophorese in bekannter
Weise aufgetrennt und die Bruchstücke durch Anfärben mit Ethidiumbromid oder durch radioaktive
Markierung ("Nick-Translation" nach Maniatis,
a.a.O.) sichtbar gemacht. Die Plasmidbande wird
anschließend aus dem Acrylamidgel ausgeschnitten
und elektrophoretisch vom Polyacrylamid abgetrennt. Die Auftrennung des Verdauungsansatzes kann
auch auf 2%igen niedrigschmelzenden Agarosegelen
(wie in Beispiel 5a) beschrieben) erfolgen.

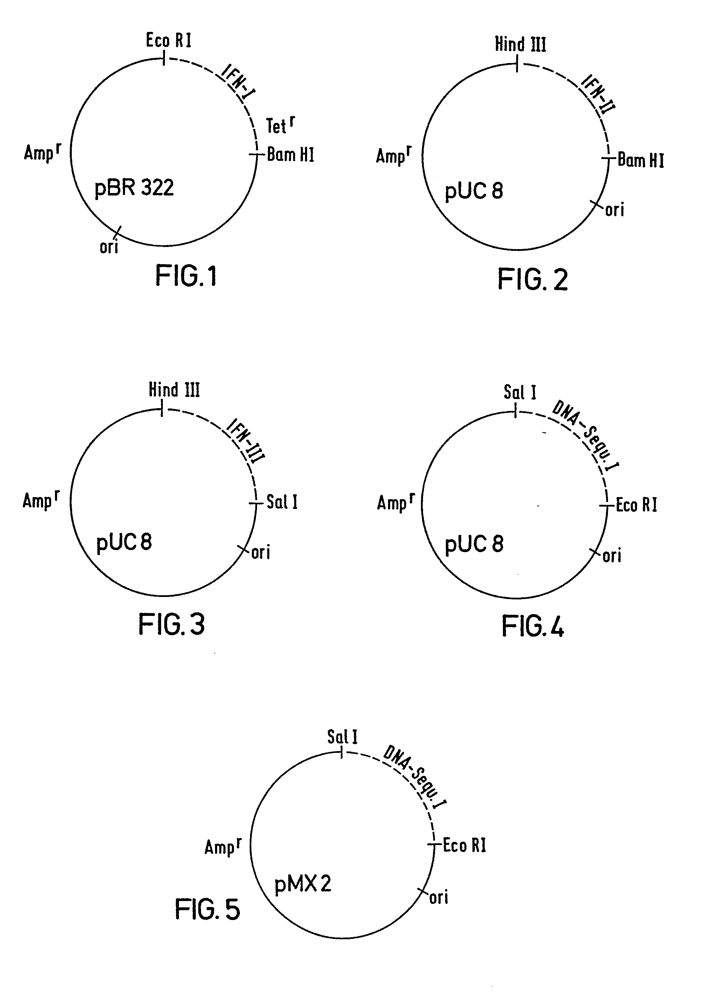

1 µg Plasmid wird dann mit 10 ng Genfragment IFN-I
über Nacht bei 16°C ligiert. Man erhält das Hybridplasmid gemäß Figur 1.

b) Einbau des Genfragments IFN-II in pUC 8
Analog zu a) wird das handelsübliche Plasmid pUC 8
mit Bam HI und Hind III aufgeschnitten und mit dem
Genfragment IFN-II ligiert. Man erhält das
Hybridplasmid gemäß Figur 2.

c) Einbau des Genfragments IFN-III in pUC 8
Analog zu a) wird das Plasmid pUC 8 mit Hind III und
Sal I aufgeschnitten und mit dem Genfragment IFN-III
ligiert. Man erhält das Hybridplasmid gemäß Figur 3.

4. Synthese des kompletten Gens und Einbau in ein Plasmid

a) Transformation und Amplifikation
Die so erhaltenen Hybridplasmide werden in E. coli
transformiert. Hierzu wird der Stamm E. coli K 12
durch Behandlung mit einer 70 mM Calciumchloridlösung
kompetent gemacht und mit der Suspension des Hybridplasmids in 10 mM Tris-HCl-Puffer (pH 7,5), der 70 mM an Calciumchlorid ist, versetzt. Die transformierten Stämme werden wie üblich unter Ausnutzung der durch das Plasmid vermittelten Antibiotikaresistenzen bzw. -empfindlichkeiten selektioniert und die Hybridvektoren amplifiziert. Nach Abtöten der Zellen werden die Hybridplasmide isoliert, mit den ursprünglich eingesetzten Restriktionsenzymen aufgeschnitten und die Genfragmente IFN-I, IFN-II und IFN-III durch Gelelektrophorese isoliert.

b) Verknüpfung der Genfragmente

Die durch Amplifikation erhaltenen Subfragmente IFN-I, IFN-II und IFN-III werden wie im Beispiel 2 beschrieben enzymatisch verknüpft und das so erhaltene synthetische Gen mit der DNA-Sequenz I in den Klonierungsvektor pUC 8 eingeführt. Man erhält ein Hybridplasmid gemäß Figur 4.

5. Konstruktion von Hybridplasmiden für die Expression der DNA-Sequenz I

a) Einbau in pKK 177.3

Das Expressionsplasmid pKK 177.3 (Plasmid ptac 11, Amman et al., Gene 25 (1983) 167, bei dem in die Eco RI-Erkennungsstelle synthetisch eine Sequenz eingebaut wurde, die eine Sal I-Schnittstelle enthält) wird mit den Restriktionsenzymen Eco RI und Sal I geöffnet. Aus dem Plasmid gemäß Figur 4 wird die Insertion I mit den Restriktionsenzymen Eco RI und Sal I herausgeschnitten.

Die Insertion I wird auf 2%ige niedrig-schmelzende (low melting) Agarose gegeben, von der Plasmid-DNA abgetrennt und die Insertion durch Auflösen des Gels bei erhöhter Temperatur (nach Maßgabe der Hersteller) wiedergewonnen. Durch Ligation des aufgeschnittenen Plasmids pKK 177.3 mit dem Gen I wird ein Hybridplasmid geschaffen, bei dem der Insertion eine Expressions bzw. Regulationsregion vorgeschaltet ist. Nach Zugabe eines geeigneten Induktors wie Isopropyl-ß-thiogalactopyranosid (IPTG) wird eine mRNA gebildet, die zur Expression des Methionyl-Polypeptids entsprechend der DNA-Sequenz I führt.

b) Einbau in pMX 2

Das Expressionsplasmid pMX 2 besteht aus einem um 21 Nucleotide verkürzten pUC 8-Plasmid und wird auf folgende Weise hergestellt:

pUC 8 wird mit der Restriktionsendonuclease Eco RI geöffnet und anschließend mit der Exonuclease Bal 31 unter Bedingungen behandelt, die die Abspaltung von etwa 20 Nucleotiden auf beiden Seiten der Eco RI-Schnittstelle erlauben (Maniatis a.a.O.). Anschließend werden eventuell überstehende Enden des so behandelten Plasmids mit Klenow-DNA-Polymerase aufgefüllt, das Plasmid mit der Restriktionsendonuclease Hind III nachgeschnitten und das Plasmid über 1%ige niedrig-schmelzende Agarosegele nach Maßgabe der Hersteller gereinigt. In das Plasmid wird der ursprünglich in pUC 8 vorhandene durch die Restriktionsenzymschnitte Eco RI und Hind III begrenzte Polylinker, der durch die zuvor beschriebenen Manipulationen zerstört wurde, reinseriert. Dazu wird pUC 8 mit dem Restriktionsenzym Eco RI geöffnet und die überstehenden Enden mit Klenow-DNA-Polymerase unter Verwendung von 32P-markierten Nucleosidtriphosphaten aufgefüllt. Der Polylinker wird anschließend aus dem Plasmid mit dem Restriktionsenzym Hind III herausgeschnitten und durch Elektrophorese auf 10% Acrylamidgelen vom Plasmid abgetrennt. Nach Identifikation der Polylinker-Bande mit Hilfe einer Autoradiographie wird der Polylinker durch Elektroelution von Acrylamidresten befreit und in das verkürzte pUC 8-Plasmid ligiert. Das so konstruierte Plasmid pMX 2 wird anschließend mit den Restriktionsenzymen Eco RI und Sal I geöffnet und mit dem γ-Interferon-Gen I, das an den Enden Eco RI- und Sal I-Erkennungssequenzen aufweist, in das Expressionsplasmid pMX 2 ligiert (Fig. 5). Klone, die einen hohen Interferontiter zeigen, werden dann anhand der biologischen Aktivitätsbestimmung identifiziert.

6. Transformation der Hybridplasmide.

Kompetente E. coli-Zellen werden mit 0,1 bis 1 µg der Hybridplasmide, die die Sequenz I enthalten, transformiert und auf Ampicillin enthaltenden Agarplatten plattiert. Anschließend lassen sich Klone, die die korrekt integrierte γ-Interferongen-Sequenz in den entsprechenden Plasmiden enthalten, durch DNA-Schnellaufarbeitung identifizieren (Maniatis a.a.O.).

7. Expression der γ-Interferon-Aktivität aufweisenden Polypeptide

Nach Transformation der genannten Hybridplasmide in E. coli wird ein Polypeptid exprimiert, das außer der γ-Interferon-Aminosäuresequenz am Aminoterminus noch eine zusätzliche Methionylgruppe trägt.

8. Aufarbeitung und Reinigung

Die zur gewünschten optischen Dichte kultivierten Bakterienstämme werden mit einem geeigneten Induktor, beispielsweise IPTG, hinreichend lange, beispielsweise 2 Stunden, inkubiert. Anschließend werden die Zellen mit 0,1 % Kresol und 0,1 mM Benzylsulfonylfluorid abgetötet. Nach Zentrifugieren oder Filtrieren wird die Zellmasse in einer Pufferlösung (50 mM Tris, 50 mM EDTA, pH 7,5) aufgenommen und mechanisch aufgeschlossen, beispielsweise mit einer French-Presse bzw. (R)DYNO-Mühle (Fa. Willy Bachofer, Basel), worauf die unlöslichen Bestandteile abzentrifugiert werden. Aus dem Überstand wird das die γ-Interferon-Aktivität enthaltende Protein nach üblichen Verfahren gereinigt. Geeignet sind Ionenaustauscher-, Adsorptions-, Gelfiltrationssäulen oder Affinitätschromatographie an Antikörpersäulen. Durch Natriumdodecylsulfat-Acrylamidgel- oder HPLC-Analytik werden Anreicherung und Reinheit des Produktes kontrolliert.

Zur biologischen Charakterisierung des Produktes auf γ-Interferonaktivität werden in bekanner Weise Indikatorzellinien, wie zum Beispiel Vero-Zellen, benutzt und mit Verdünnungsreihen interferonhaltiger Bakterienextrakte inkubiert. Anschließend wird durch Infektion mit einem Virus wie VSV (Vesicular Stomatitis Virus) überprüft, bis zu welchem Verdünnungsschritt die Vorbehandlung mit dem Bakterienextrakt bei den Vero-Zellen ein antiviraler Status erzielt werden konnte. Die Auswertung kann mikroskopisch oder durch Bestimmung der Neutralrot-Aufnahme erfolgen.

Die γ-Interferon-Aktivität kann auch mit einem handelsüblichen Radioimmunoassay (Celltech Ltd.), der auf einem monoklonalen Antikörper gegen γ-Interferon aufgebaut ist, erfolgen.

DNA–Sequenz I

| Triplett Nr. | | | | | 1 | 2 | 3 | 4 | 5 |
|---|---|---|---|---|---|---|---|---|---|
| Aminosäure | | | | | Cys | Tyr | Cys | Gln | Asp |
| Nucleotid Nr. | | | | | | | | 10 | |
| Cod. Strang | 5' | | | | TGC | TAC | TGC | CAG | GAC |
| nicht cod. Strang | 3' | | | | ACG | ATG | ACG | GTC | CTG |

| 6 | 7 | 8 | 9 | 10 | 11 | 12 | 13 | 14 | 15 |
|---|---|---|---|---|---|---|---|---|---|
| Pro | Tyr | Val | Lys | Glu | Ala | Glu | Asn | Leu | Lys |
| | 20 | | | 30 | | | | 40 | |
| CCG | TAC | GTT | AAA | GAA | GCT | GAA | AAC | CTG | AAA |
| GGC | ATG | CAA | TTT | CTT | CGA | CTT | TTG | GAC | TTT |

| 16 | 17 | 18 | 19 | 20 | 21 | 22 | 23 | 24 | 25 |
|---|---|---|---|---|---|---|---|---|---|
| Lys | Tyr | Phe | Asn | Ala | Gly | His | Ser | Asp | Val |
| | 50 | | | 60 | | | | 70 | |
| AAA | TAC | TTC | AAC | GCT | GGT | CAT | TCT | GAC | GTT |
| TTT | ATG | AAG | TTG | CGA | CCA | GTA | AGA | CTG | CAA |

| 26 | 27 | 28 | 29 | 30 | 31 | 32 | 33 | 34 | 35 |
|---|---|---|---|---|---|---|---|---|---|
| Ala | Asp | Asn | Gly | Thr | Leu | Phe | Leu | Gly | Ile |
| | 80 | | | 90 | | | | 100 | |
| GCT | GAC | AAT | GGT | ACT | CTG | TTC | CTG | GGG | ATC |
| CGA | CTG | TTA | CCA | TGA | GAC | AAG | GAC | CCC | TAG |

| 36 | 37 | 38 | 39 | 40 | 41 | 42 | 43 | 44 | 45 |
|---|---|---|---|---|---|---|---|---|---|
| Leu | Lys | Asn | Trp | Lys | Glu | Glu | Ser | Asp | Arg |
| | 110 | | | 120 | | | | 130 | |
| CTG | AAA | AAC | TGG | AAA | GAA | GAA | TCT | GAC | CGT |
| GAC | TTT | TTG | ACC | TTT | CTT | CTT | AGA | CTG | GCA |

| 46 | 47 | 48 | 49 | 50 | 51 | 52 | 53 | 54 | 55 |
|---|---|---|---|---|---|---|---|---|---|
| Lys | Ile | Met | Gln | Ser | Gln | Ile | Val | Ser | Phe |
|  | 140 |  |  | 150 |  |  |  | 160 |  |
| AAA | ATC | ATG | CAA | TCT | CAG | ATC | GTT | TCT | TTC |
| TTT | TAG | TAC | GTT | AGA | GTC | TAG | CAA | AGA | AAG |

| 56 | 57 | 58 | 59 | 60 | 61 | 62 | 63 | 64 | 65 |
|---|---|---|---|---|---|---|---|---|---|
| Tyr | Phe | Lys | Leu | Phe | Lys | Asn | Phe | Lys | Asp |
|  | 170 |  |  | 180 |  |  |  | 190 |  |
| TAC | TTC | AAA | CTG | TTC | AAA | AAC | TTT | AAA | GAC |
| ATG | AAG | TTT | GAC | AAG | TTT | TTG | AAA | TTT | CTG |

| 66 | 67 | 68 | 69 | 70 | 71 | 72 | 73 | 74 | 75 |
|---|---|---|---|---|---|---|---|---|---|
| Asp | Gln | Ser | Ile | Gln | Lys | Ser | Val | Glu | Thr |
|  | 200 |  |  | 210 |  |  |  | 220 |  |
| GAC | CAG | TCT | ATC | CAG | AAA | TCT | GTT | GAA | ACT |
| CTG | GTC | AGA | TAG | GTC | TTT | AGA | CAA | CTT | TGA |

| 76 | 77 | 78 | 79 | 80 | 81 | 82 | 83 | 84 | 85 |
|---|---|---|---|---|---|---|---|---|---|
| Ile | Lys | Glu | Asp | Met | Asn | Val | Lys | Phe | Phe |
|  | 230 |  |  | 240 |  |  |  | 250 |  |
| ATC | AAG | GAA | GAC | ATG | AAC | GTT | AAA | TTT | TTC |
| TAG | TTC | CTT | CTG | TAC | TTG | CAA | TTT | AAA | AAG |

| 86 | 87 | 88 | 89 | 90 | 91 | 92 | 93 | 94 | 95 |
|---|---|---|---|---|---|---|---|---|---|
| Asn | Ser | Asn | Lys | Lys | Lys | Arg | Asp | Asp | Phe |
|  | 260 |  |  | 270 |  |  |  | 280 |  |
| AAC | TCT | AAC | AAA | AAA | AAA | CGT | GAC | GAC | TTC |
| TTG | AGA | TTG | TTT | TTT | TTT | GCA | CTG | CTG | AAG |

| 96 | 97 | 98 | 99 | 100 | 101 | 102 | 103 | 104 | 105 |
|---|---|---|---|---|---|---|---|---|---|
| Glu | Lys | Leu | Thr | Asn | Tyr | Ser | Val | Thr | Asp |
|  | 290 |  |  | 300 |  |  |  | 310 |  |
| GAA | AAG | CTT | ACT | AAC | TAC | TCT | GTT | ACT | GAT |
| CTT | TTC | GAA | TGA | TTG | ATG | AGA | CAA | TGA | CTA |

| 106 | 107 | 108 | 109 | 110 | 111 | 112 | 113 | 114 | 115 |
|-----|-----|-----|-----|-----|-----|-----|-----|-----|-----|
| Leu | Asn | Val | Gln | Arg | Lys | Ala | Ile | His | Glu |
|     | 320 |     |     | 330 |     |     |     | 340 |     |
| TTA | AAC | GTT | CAA | CGT | AAA | GCT | ATC | CAC | GAG |
| AAT | TTG | CAA | GTT | GCA | TTT | CGA | TAG | GTG | CTC |

| 116 | 117 | 118 | 119 | 120 | 121 | 122 | 123 | 124 | 125 |
|-----|-----|-----|-----|-----|-----|-----|-----|-----|-----|
| Leu | Ile | Gln | Val | Met | Ala | Glu | Leu | Ser | Pro |
|     | 350 |     |     | 360 |     |     |     | 370 |     |
| CTC | ATC | CAG | GTT | ATG | GCT | GAA | CTG | TCT | CCT |
| GAG | TAG | GTC | CAA | TAC | CGA | CTT | GAC | AGA | GGA |

| 126 | 127 | 128 | 129 | 130 | 131 | 132 | 133 | 134 | 135 |
|-----|-----|-----|-----|-----|-----|-----|-----|-----|-----|
| Ala | Ala | Lys | Thr | Gly | Lys | Arg | Lys | Arg | Ser |
|     | 380 |     |     | 390 |     |     |     | 400 |     |
| GCA | GCT | AAA | ACT | GGT | AAA | CGT | AAA | CGT | TCC |
| CGT | CGA | TTT | TGA | CCA | TTT | GCA | TTT | GCA | AGG |

| 136 | 137 | 138 | 139 | 140 | 141 | 142 | 143 | 144 | 145 |
|-----|-----|-----|-----|-----|-----|-----|-----|-----|-----|
| Gln | Met | Leu | Phe | Arg | Gly | Arg | Arg | Ala | Ser |
|     | 410 |     |     | 420 |     |     |     | 430 |     |
| CAG | ATG | CTG | TTC | CGC | GGT | CGT | CGT | GCT | TCT |
| GTC | TAC | GAC | AAG | GCG | CCA | GCA | GCA | CGA | AGA |

| 146 |
|-----|
| Gln |
| 438 |
| CAG | 3' |
| GTC | 5' |

DNA-Sequenz II

IFN-I:

|  |  | Met | Cys | Tyr | Cys | Gln | Asp |
|---|---|---|---|---|---|---|---|
| 5' AA | TTC | ATG | TGC | TAC | TGC | CAG | GAC |
| 3' | G | TAC | ACG | ATG | ACG | GTC | CTG |

Eco RI

← Ia →

← Ib ←

| Pro | Tyr | Val | Lys | Glu | Ala | Glu | Asn | Leu | Lys |
|---|---|---|---|---|---|---|---|---|---|
| CCG | TAC | GTT | AAA | GAA | GCT | GAA | AAC | CTG | AAA |
| GGC | ATG | CAA | TTT | CTT | CGA | CTT | TTG | GAC | TTT |

← Ic →

Id

| Lys | Tyr | Phe | Asn | Ala | Gly | His | Ser | Asp | Val |
|---|---|---|---|---|---|---|---|---|---|
| AAA | TAC | TTC | AAC | GCT | GGT | CAT | TCT | GAC | GTT |
| TTT | ATG | AAG | TTG | CGA | CCA | GTA | AGA | CTG | CAA |

Ie →

Ig

If

Ih

Ii

| Ala | Asp | Asn | Gly | Thr | Leu | Phe | Leu | (Gly) |
|---|---|---|---|---|---|---|---|---|
| GCT | GAC | AAT | GGT | ACT | CTG | TTC | CTG | GG |
| CGA | CTG | TTA | CCA | TGA | GAC | AAG | GAC | CCC | TAG |

Ij

Bam HI

0155590

DNA-Sequenz II

IFN-II:

```
←─────────────────────────── IIa ──────────────────────────→│  ←

(Gly) Ile  Leu    Lys      Asn      Trp    Lys    Glu    Glu    Ser    Asp │ Arg
   G  ATC  CTG    AAA      AAC      TGG    AAA    GAA    GAA    TCT    GAC │ CGT
Bam HI     GAC    TTT      TTG      ACC    TTT    CTT    CTT    AGA    CTG │ GCA
         ←───────────────────────── IIb ─────────────────────────────────
  ─────────────────────────────── IIc ───────────────────────────────→│
 Lys      Ile      Met  . Gln     Ser    Gln    Ile     Val    Ser    Phe │
 AAA      ATC      ATG    CAA     TCT    CAG    ATC     GTT    TCT    TTC │
 TTT│     TAG      TAC    GTT     AGA    GTC    TAG     CAA    AGA    AAG │
 ───→│ ←──────────────────────── IId ─────────────────────────────────
   │←─────────────────────────── IIe ───────────────────────────────→│
 Tyr      Phe      Lys    Leu  .  Phe    Lys    Asn    Phe     Lys    Asp │
 TAC      TTC      AAA    CTG     TTC    AAA    AAC    TTT     AAA    GAC │
 ATG      AAG│     TTT    GAC     AAG    TTT    TTG    AAA     TTT    CTG │
 ──────────→│ ←──────────────────── IIf ──────────────────────────────
   │←───────────────────────── IIg ───────────────────────────────
 Asp      Gln      Ser    Ile    Gln    Lys    Ser    Val    Glu    Thr
 GAC      CAG      TCT    ATC    CAG    AAA    TCT    GTT    GAA    ACT
 CTG      GTC│     AGA    TAG    GTC    TTT    AGA    CAA    CTT    TGA
 ──────────→│ ←──────────────────── IIh ──────────────────────────────
   │←│←───────────────────────── IIi ──────────────────────────────
 Ile│     Lys      Glu    Asp    Met    Asn    Val    Lys    Phe    Phe
 ATC│     AAG      GAA    GAC    ATG    AAC    GTT    AAA    TTT    TTC
 TAG│     TTC      CTT│   CTG    TAC    TTG    CAA    TTT.   AAA    AAG
 ──────→│ ←────────────────────── IIj ──────────────────────────────
   │←│←──────────────────────── IIk ──────────────────────────────
 Asn│     Ser      Asn    Lys    Lys    Lys    Arg    Asp    Asp .  Phe
 AAC│     TCT      AAC    AAA    AAA    AAA    CGT    GAC    GAC    TTC
 TTG│     AGA      TTG    TTT    TTT    TTT    GCA    CTG    CTG    AAG
 ──────→│ ←──────────────────────── IIl ──────────────────────────────
 Glu      (Lys)

 GAA      A

 CTT      TTC      GA     Hind III
                  ──→
  ──────────────────────────────────────────────────────────────────
```

DNA-Sequenz II

IFN-III:

|  | ←——————————————————————— IIIa ———————————————————→ | | | | | | | |
|---|---|---|---|---|---|---|---|---|
|  | (Lys) | Leu | Thr | Asn | Tyr | Ser | Val | Thr | Asp |
|  | . AG | CTT | ACT | AAC | TAC | TCT | GTT | ACT | GAT |
| Hind III | | A | TGA | TTG | ATG | AGA | CAA | TGA | CTA |

← ——————————— IIIb ———————————

——————————————— IIIc ———————————————→ ←——————————————

| Leu | Asn | Val | Gln | Arg | Lys | Ala | Ile | His | Glu |
|---|---|---|---|---|---|---|---|---|---|
| TTA | AAC | GTT | CAA | CGT | AAA | GCT | ATC | CAC | GAG |
| AAT | TTG | CAA | GTT | GCA | TTT | CGA | TAG | GTG | CTC |

←———————————— IIId ————————————————————→

——————————————— IIIe ———————————————→ ←——————————————

| Leu | Ile | Gln | Val | Met | Ala | Glu | Leu | Ser | Pro |
|---|---|---|---|---|---|---|---|---|---|
| CTC | ATC | CAG | GTT | ATG | GCT | GAA | CTG | TCT | CCT |
| GAG | TAG | GTC | CAA | TAC | CGA | CTT | GAC | AGA | GGA |

←———————————— IIIf ————————————————————→ ←

——————————— IIIg ———————————→ ←——— IIIi ———

| Ala | Ala | Lys | Thr | Gly | Lys | Arg | Lys | Arg | Ser |
|---|---|---|---|---|---|---|---|---|---|
| GCA | GCT | AAA | ACT | GGT | AAA | CGT | AAA | CGT | TCC |
| CGT | CGA | TTT | TGA | CCA | TTT | GCA | TTT | GCA | AGG |

——————————————— IIIh ———————————————→ ←

—— IIIi ———————→ ←——————— IIIk ———

| Gln | Met | Leu | Phe | Arg | Gly | Arg | Arg | Ala | Ser |
|---|---|---|---|---|---|---|---|---|---|
| CAG | ATG | CTG | TTC | CGC | GGT | CGT | CGT | GCT | TCT |
| GTC | TAC | GAC | AAG | GCG | CCA | GCA | GCA | CGA | AGA |

————————— IIIj ———————————————→ ←——————— IIIl ———

———————————→

| Gln | Stp | Stp | | |
|---|---|---|---|---|
| CAG | TAA | TAG | | 3' |
| GTC | ATT | ATC | AGC T | 5' |

————————————————————→

Sal I

<u>PATENTANSPRÜCHE:</u>

1. Gentechnologisches Verfahren zur Herstellung von Human-Gammainterferon, dadurch gekennzeichnet, daß ein synthetisches Gen eingesetzt wird, das die DNA-Sequenz I enthält.

2. Verfahren nach Anspruch 1, dadurch gekennzeichnet, daß im Anschluß an das Gen zwei Stop-Codons folgen.

3. Verfahren nach Anspruch 1 oder 2, dadurch gekennzeichnet, daß als Wirtsorganismus E. coli dient.

4. DNA-Sequenzen I, IIa (IFN-I), IIb (IFN-II) und IIc (IFN-III).

5. DNA-Oligonucleotide Ia bis IIIl.

6. Hybridplasmide, die zwischen einer Eco RI- und einer Bam HI-Schnittstelle die DNA-Sequenz IIa (IFN-I), zwischen einer Bam HI- und einer Hind III-Schnittstelle die DNA-Sequenz IIb (IFN-II), zwischen einer Hind III- und einer Sal I-Schnittstelle die DNA-Sequenz IIc (IFN III) oder zwischen einer Eco RI- und einer Sal I-Schnittstelle die DNA-Sequenz I enthalten.

7. Hybridplasmide nach Anspruch 6, die zwischen der Eco RI-Schnittstelle und dem Codon für die erste Aminosäure Cystein eine Praesequenz eines sekretorischen Proteins im richtigen Leserahmen enthalten.

8. Wirtsorganismen, die Hybridplasmide nach Anspruch 6 oder 7 enthalten.

9. E. coli, enthaltend ein Hybridplasmid nach Anspruch 6 oder 7.

Patentansprüche Österreich:

1. Gentechnologisches Verfahren zur Herstellung von Human-Gammainterferon, dadurch gekennzeichnet, daß ein synthetisches Gen eingesetzt wird, das die DNA-Sequenz I enthält.

2. Verfahren nach Anspruch 1, dadurch gekennzeichnet, daß im Anschluß an das Gen zwei Stop-Codons folgen.

3. Verfahren nach Anspruch 1 oder 2, dadurch gekennzeichnet, daß als Wirtsorganismus E. coli dient.

1/1

0155590

FIG.1

FIG. 2

FIG.3

FIG.4

FIG. 5